# EUROPEAN PATENT SPECIFICATION

(11) **EP 4 616 071 B1**
(45) Date of publication and mention of the grant of the patent: **18.03.2026**
(21) Application number: 23800355.2
(22) Date of filing: 30.10.2023
(51) Int. Cl.: F04B 43/04, A61B 6/00, F04B 41/06, F04B 45/04, F04B 23/06

(54) **MULTI-ORIENTATION OMNIDIRECTIONAL PUMP SYSTEMS FOR USE IN MAGNETIC RESONANCE ENVIRONMENTS**
OMNIDIREKTIONALE PUMPENSYSTEME MIT MEHREREN AUSRICHTUNGEN ZUR VERWENDUNG IN MAGNETRESONANZUMGEBUNGEN
SYSTÈMES DE POMPE OMNIDIRECTIONNELLE MULTI-ORIENTATION DESTINÉS À ÊTRE UTILISÉS DANS DES ENVIRONNEMENTS DE RÉSONANCE MAGNÉTIQUE

(30) Priority: 09.11.2022 US 202263423924 P
(43) Date of publication of application: 17.09.2025
(73) Proprietor: Koninklijke Philips N.V., 5656 AG Eindhoven (NL)
(72) Inventor: KOSTAKIS, Dimitri George, 5656 AG Eindhoven (NL); YEINI, Avraham, 5656 AG Eindhoven (NL); BROWN, Richard Earl, 5656 AG Eindhoven (NL)
(74) Representative: Philips Intellectual Property & Standards
(86) International application number: PCT/EP2023/080195
(87) International publication number: WO 2024/099810

(56) References cited:
- EP-A1- 3 501 389
- US-A1- 2006 013 710
- US-A1- 2011 227 237
- US-B2- 10 648 463

## Description

### Field of the Disclosure

The present disclosure relates generally to fluid flow systems and methods, and more specifically to omnidirectional fluid flow systems and methods utilizing multi-orientation fluid flow pumps.

### Background

Fluid pumps are used alone or in combination with other pumps in a fluid flow system to achieve a desired flow rate and/or pressure curve within a required amount of time. However, depending on the intended application of the fluid flow system, certain limitations may be placed on the types of fluid pumps and/or materials used to construct the fluid flow system. For example, when used in medical devices, these limitations can include safety- and/or performance-related requirements, such as minimum- or maximum-rated pressures, flowrates, voltage and current specifications, and the like. These limitations can also include an operability requirement, such as requiring that the fluid flow system operate in environments subjected to high magnetic fields (e.g., in a magnetic resonance imaging suite, etc.). While conventional pumps may meet many of the performance-related requirements, these conventional pumps contain materials and/or mechanisms that are negatively affected by high magnetic fields, which negatively impacts the performance of the pump systems and potentially makes them unacceptable for use in certain medical devices. EP3501389A1 relates to apparatuses for pumping fluid (e.g., gas or liquid) in a magnetic resonance image (MRI) environment of high magnetic fields and required low radiofrequency interference.

### Summary of the Disclosure

According to an embodiment of the present disclosure, an omnidirectional pump system for use in a magnetic resonance environment is provided. The pump system comprises: a first pump configured to communicate a first fluid flow from a fluid source to an associated medical device, the first pump including a conductive element having a first plane; a second pump configured to communicate a second fluid flow from the fluid source to the medical associated device with the first pump, the second pump including a conductive element having a second plane; wherein the first pump and the second pump are arranged such that the first plane and the second plane are not simultaneously parallel to an associated magnetic field in proximity to the omnidirectional pump system.

In an aspect, the first pump and the second pump are arranged such that the first plane and the second plane cannot be not simultaneously be parallel to an associated magnetic field in proximity to the omnidirectional pump system.

In an aspect, the associated magnetic field in proximity to the omnidirectional pump system is perpendicular to at least one of the first plane of the conductive element of the first pump and the second plane of the conductive element of the second pump.

In an aspect, the associated magnetic field has a magnetic flux density of at least 1,000 gauss, and wherein the first pump and the second pump are rated for use in the associated magnetic field.

In an aspect, the omnidirectional pump system further comprises a third pump configured to communicate a third fluid flow from the fluid source to the associated medical device with the first and second pumps, the third pump including a conductive element having a third plane, wherein the first, second, and third pumps are arranged such that the first, second, and third planes are not simultaneously parallel to the associated magnetic field in proximity to the omnidirectional pump system.

In an aspect, the first pump is at least one of a rolling magnet pump, a stator rotation pump, and a piezoelectric pump; and wherein the second pump is at least one of a rolling magnet pump, a stator rotation pump, and a piezoelectric pump.

In an aspect, the associated medical device is a non-invasive blood pressure cuff, and the first and second fluid flows comprise air.

In an aspect, the associated medical device is a respiratory mask, and at least one of the first and second fluid flows comprises an anesthetic agent.

In an aspect, the omnidirectional pump system is movable relative to the associated magnetic field while the first plane of the conductive element of the first pump is fixed relative to the second plane of the conductive element of the second pump.

In an aspect, each of the conductive elements are actuated during operation of the corresponding pump.

According to another embodiment of the present disclosure, a medical apparatus is provided. The medical apparatus comprises an omnidirectional pump system configured to produce a fluid output and a patient-worn device configured to receive the fluid output, wherein the patient-worn device is coupled to a patient. In an aspect, the omnidirectional pump system includes: a first pump configured to communicate a first fluid flow from a fluid source to the patient-worn device, the first pump including a conductive element having a first plane; and a second pump configured to communicate a second fluid flow from the fluid source to the patient-worn device with the first pump, the second pump including a conductive element having a second plane. In an aspect, the first pump and the second pump are arranged such that the first plane and the second plane are not simultaneously parallel to an associated magnetic field in proximity to the omnidirectional pump system.

In an aspect, the medical apparatus is at least one of: an MRI-compatible medical monitor; an anesthesia cart; a bedside monitor; a non-invasive blood pressure monitor; and a non-invasive gas monitor.

According to another embodiment of the present disclosure, a method of regulating delivery of a fluid flow to a medical device coupled to a patient is provided. The method comprises: flowing a first fluid input though at least a first pump of an omnidirectional pump system along a first fluid pathway to the medical device; and flowing a second fluid input through at least a second pump of the omnidirectional pump system along a second fluid pathway to the medical device; wherein the first pump includes a conductive element having a first plane, the second pump includes a conductive element having a second plane, and the first pump and second pump are arranged such that the first plane and the second plane are not simultaneously parallel to an associated magnetic field in proximity to the medical device.

In an aspect, the omnidirectional pump system is movable relative to the associated magnetic field while the first plane of the conductive element of the first pump is fixed relative to the second plane of the conductive element of the second pump.

In an aspect, the first plane is defined by the longest straight-line distance through the conductive element of the first pump, and the second plane is defined by the longest straight-line distance through the conductive element of the second pump.

These and other aspects of the various embodiments will be apparent from and elucidated with reference to the embodiments described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a diagram of patient in an environment subject to a high magnetic field illustrated according to aspects of the present disclosure.
FIG. 2 is a diagram of an individual fluid flow pump illustrated according to aspects of the present disclosure.
FIG. 3A is a side view of a conductive element in a first orientation illustrated according to aspects of the present disclosure.
FIG. 3B is a top view of the conductive element in the first orientation illustrated according to aspects of the present disclosure.
FIG. 3C is a side view of the conductive element in a second orientation illustrated according to aspects of the present disclosure.
FIG. 3D is a side view of the conductive element in a third orientation illustrated according to aspects of the present disclosure.
FIG. 4 is a graph showing the performance of a single pump based on its orientation relative to a nearby magnetic field according to aspects of the present disclosure.
FIG. 5A is a block diagram of an omnidirectional pump system illustrated according to aspects of the present disclosure.
FIG. 5B is another block diagram of an omnidirectional pump system illustrated according to aspects of the present disclosure.
FIG. 6 is a perspective illustration showing conductive elements having different orientations relative to a nearby magnetic field according to aspects of the present disclosure.
FIG. 7 is an illustration showing a medical apparatus including an omnidirectional pump system according to aspects of the present disclosure.
FIG. 8 is a flowchart of a method for regulating delivery of a fluid flow to a medical apparatus coupled to a patient illustrated according to aspects of the present disclosure.

### Detailed Description of Embodiments

The present disclosure is directed to omnidirectional fluid flow systems and methods adapted for use in specialized environments, such as environments subjected to high magnetic fields. In particular, it is appreciated by the present disclosure that, among other drawbacks, Eddy currents can be generated within conventional fluid flow pump systems when utilized in environments subjected to high magnetic fields.

As used herein, the term "Eddy current" refers to a swirling of a fluid flow and/or a reverse current created in opposition to a primary and intended fluid flow. It has been found that such Eddy currents can negatively impact different safety- and/or performance-related specifications of such pump systems.

It has also been found that the creation of such Eddy currents can depend on the orientation of the pump or a conductive element within the pump itself. Accordingly, the present disclosure is directed to systems and methods that utilize two or more fluid flow pumps arranged in multiple orientations relative to a nearby magnetic field.

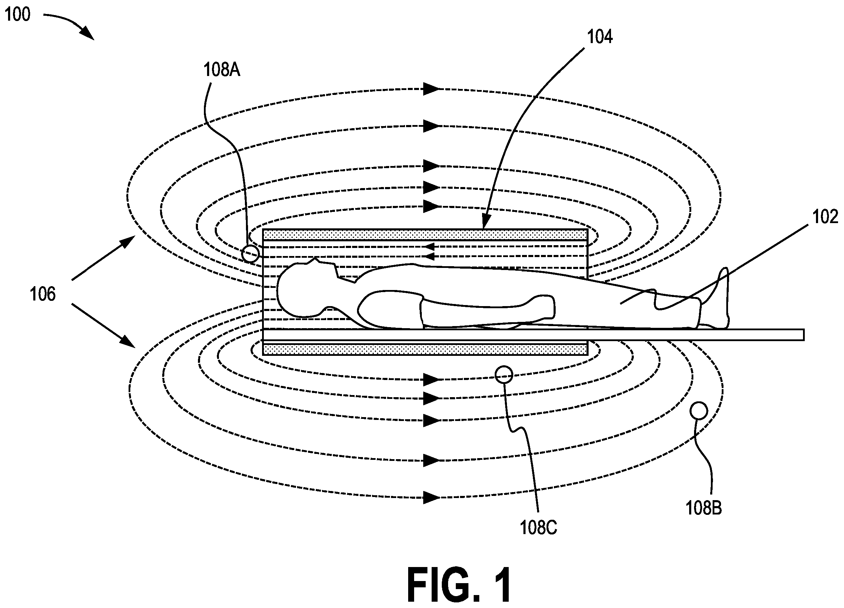

With reference FIG. 1, an environment 100 that is subjected to high magnetic fields is illustrated according to aspects of the present disclosure. As shown, a subject 102 positioned within proximity to a magnetic field source 104 that generates an associated magnetic field 106. In embodiments, the magnetic field source 104 can be a magnetic resonance imaging (MRI) machine, however, other magnetic field sources 104 are contemplated. The subject 102 may be located within the magnetic field source 104 itself (e.g., in the case of an MRI machine), or may be located nearby to the magnetic field source 104 but within the magnetic field 106 generated by the magnetic field source 104.

According to various embodiments, the magnetic field source 104 may produce an associated magnetic field 106 (sometimes symbolized as *̅B̅*̅), having a direction relative to the magnetic field source 104 and/or the environment 100 subjected to the magnetic field 106. In further embodiments, the magnetic field 106 may have a direction relative to the subject 102 and/or any medical apparatuses within the environment 100.

Additionally, the subject 102 and/or any medical apparatuses within the environment 100 may further experience the associated magnetic field 106 having a field strength (e.g., magnetic flux density). For example, in some embodiments, the associated magnetic field 106 may have a magnetic flux density that is greater than about 1000 gauss (about 0.1 tesla), including greater or equal to than about 5,000 gauss (i.e., about 0.5 tesla), greater than or equal to about 10,000 gauss (i.e., about 1 tesla), and greater than or equal to about 15,000 gauss (i.e., about 1.5 tesla).

In embodiments, the direction and/or the strength of the magnetic field 106 may vary depending on the location-of-interest within the environment 100. For example, the associated magnetic field 106 can have a first direction and/or a first strength at a first location 108A within the environment 100, can have a second direction and/or a second strength at a second location 108B within the environment 100, can have a third direction and/or a third strength at a third location 108C, and so on. As such, when moving from a first location (e.g., location 108A) to a second location (e.g., location 108B) and/or one or more other locations (e.g., location 108C), the subject 102 and/or any medical apparatuses may experience a change in the direction and/or the strength of the magnetic field 106.

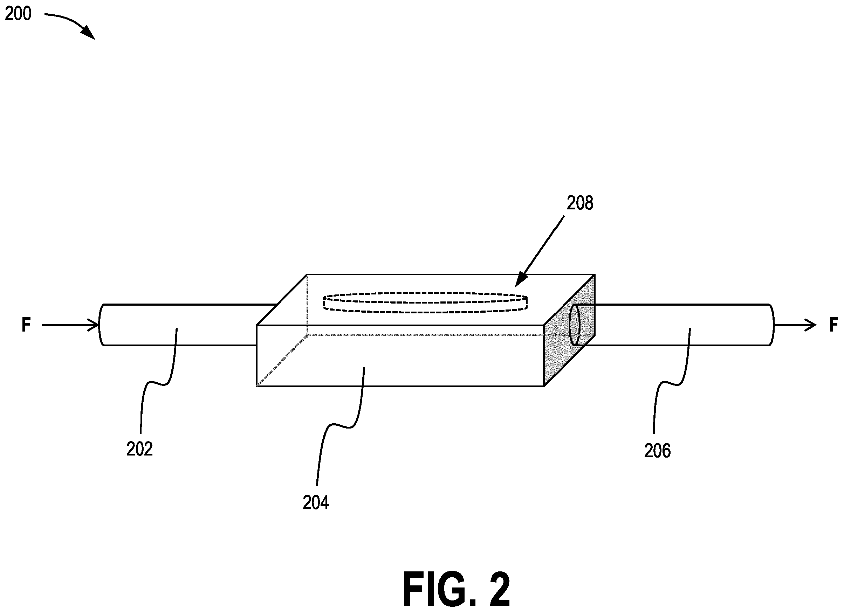

Turning to FIG. 2, an illustration of a fluid flow pump 200 that can be used in a variety of medical apparatuses within an environment 100 subjected to high magnetic fields is provided according to aspects of the present disclosure. As shown, the fluid flow pump 200 can include a fluid flow input conduit 202, a pump housing or body 204, and a fluid flow output conduit 206. A fluid flow 'F' may be communicated to the pump body 204 from a fluid flow source (not shown) via the input conduit 202, and may be communicated to a medical device (not shown) via the output conduit 206.

In embodiments, the pump 200 may be configured to deliver the fluid flow 'F' at a fixed and/or variable flow rate (e.g., between about 1 mL/min to about 50 mL/min, etc.). In some embodiments, the pump 200 includes a conductive element 208 disposed within the pump body 204. In particular embodiments, the conductive element 208 is coupled to an actuator configured to produce at fluid flow 'F' having the fixed or variable flow rate.

For example, the conductive element 208 can be formed from a paramagnetic alloy, such as alloys comprising aluminum and/or titanium. In some embodiments, the conductive element 208 is not ferromagnetic. In further embodiments, the conductive element 208 may be, for example and without limitation, a piezoelectric actuator. In some embodiments, the conductive element 208 may be a component coupled to the piezoelectric actuator, such as a metallic substrate and/or a support layer.

The conductive element 208 may have different shapes, sizes, and/or dimensions based on the particular application or design of the pump 200. In embodiments, the shape, size, and/or dimensions of the conductive element 208 may define at least a first plane and a second plane. As described herein, the relationship between the first and/or second planes of the conductive element 208 relative to the local magnetic field 106 is referred to as the "orientation" of the conductive element 208.

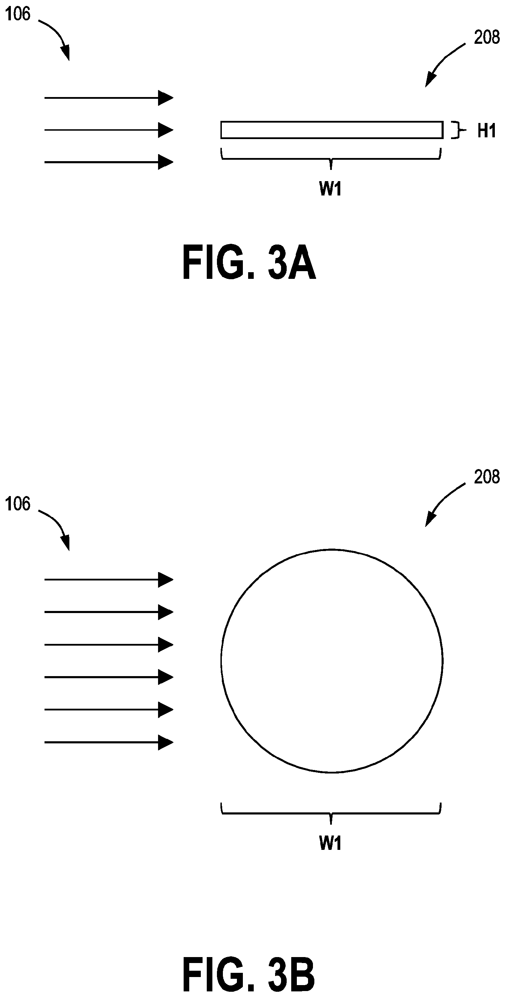

For example, with reference to FIGS. 3A and 3B, a conductive element 208 of a fluid flow pump 200 is illustrated from a side perspective (FIG. 3A) and from a top perspective (FIG. 3B). The conductive element 208 can have a first plane defined by a height 'H1' and a second plane defined by a diameter 'W1'. In the example of FIG. 3A and FIG. 3B, the second plane 'W1' is a maximal (i.e., longest) plane of the conductive element 208 and the first plane is a minimal (i.e., shortest) plane of the conductive element 208. That is, as shown, the longest straight-line distance across the conductive element 208 lays in the second plane while the shortest straight-line distance across the conductive element 208 lays in the first plane. Put another way, the conductive element 208 shown in FIGS. 3A and 3B has an orientation such that the direction of the local magnetic field 106 is parallel to the second plane 'W1' (i.e., longest plane) of the conductive element 208, and is perpendicular to the first plane 'H1' (i.e., shortest plane) of the conductive element 208.

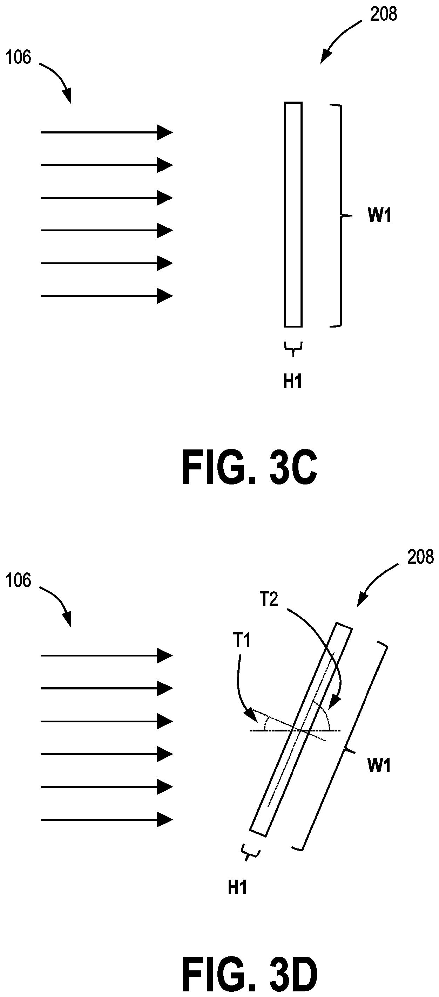

In embodiments, the orientation of the pump 200 and/or the conductive element 208 relative to the direction of the magnetic field 106 may vary. For example, with reference to FIGS. 3C and 3D, the conductive element 208 is illustrated from a side perspective in two additional orientations. As shown in FIG. 3C, the conductive element 208 has an orientation such that the direction of the local magnetic field 106 is perpendicular to the second plane 'W1' and is parallel to the first plane 'H1'. As shown in FIG. 3D, the conductive element 208 has an orientation such that the direction of the local magnetic field 106 is angled relative to the first plane 'H1' and the second plane 'W1'. In particular, angles T1 and T2 are defined between the direction of the magnetic field 106 and the first and second planes, respectively. In embodiments, each of the angles T1, T2 formed can independently be from about 0° to about 90°, including about 15°, about 30°, about 45°, about 60°, and/or about 75°.

Although the conductive element 208 is shown in FIGS. 3A-3D as a cylinder or as having a disc-shape, it is contemplated that the conductive element 208 can take other shapes, such as rectangular and/or square. In embodiments, the conductive element 208 has a first plane and a first orientation relative to an associated magnetic field, and a second plane and a second orientation relative to the associated magnetic field that is different from the first plane and first orientation. More specifically, in embodiments, the conductive element 208 can have at least a first plane defined by the shortest straight-line distance through the conductive element 208, and at least a second plane defined by the longest straight-line distance through the conductive element 208.

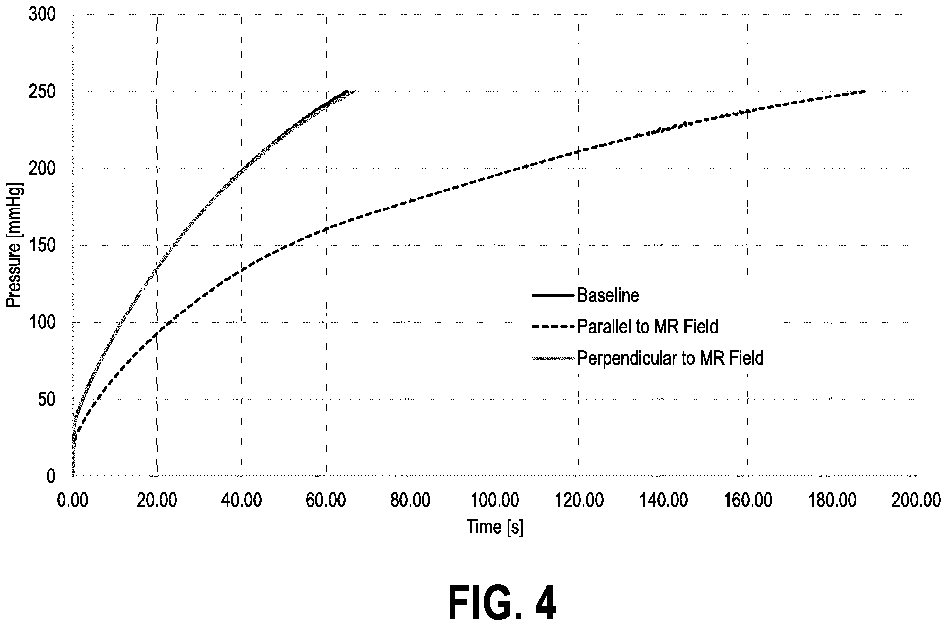

As mentioned above, the creation of Eddy currents due to the orientation of the pump 200 and/or conductive element 208 relative to the direction of a strong magnetic field 106 can negatively impact different safety- and/or performance-related specifications of such pump systems. For example, with reference to FIG. 4, a plot of the performance of a fluid flow pump (e.g., pump 200) in three trials is illustrated. In a baseline trial where the pump is not subjected to a magnetic field (e.g., magnetic field 106), the pump can produce a pressure of 250 mmHg in an associated medical device within about 65 seconds.

In a second trial where the pump 200 is oriented such that the maximal (i.e., longest) plane of the conductive element 208 within the pump 200 is parallel to the local magnetic field 106, the performance of the pump 200 is impaired such that it takes over 180 seconds to achieve the same amount of pressure in the associated medical device (i.e., over 2.5 times the amount of time to achieve the same pressure level). This trial corresponds to, for example, the orientation depicted in FIGS. 3A and 3B.

However, as shown in a third trial where the pump 200 is oriented such that the direction of the local magnetic field 106 is perpendicular to the maximal (i.e., longest) plane of the conductive element 208 and parallel to a minimal (i.e., shortest) plane of the conductive element 208, the performance of the pump 200 is substantially similar to the baseline trial and produces a pressure about 250 mmHg in the associated medical device within about 67 seconds (i.e., a difference of less than 5%).

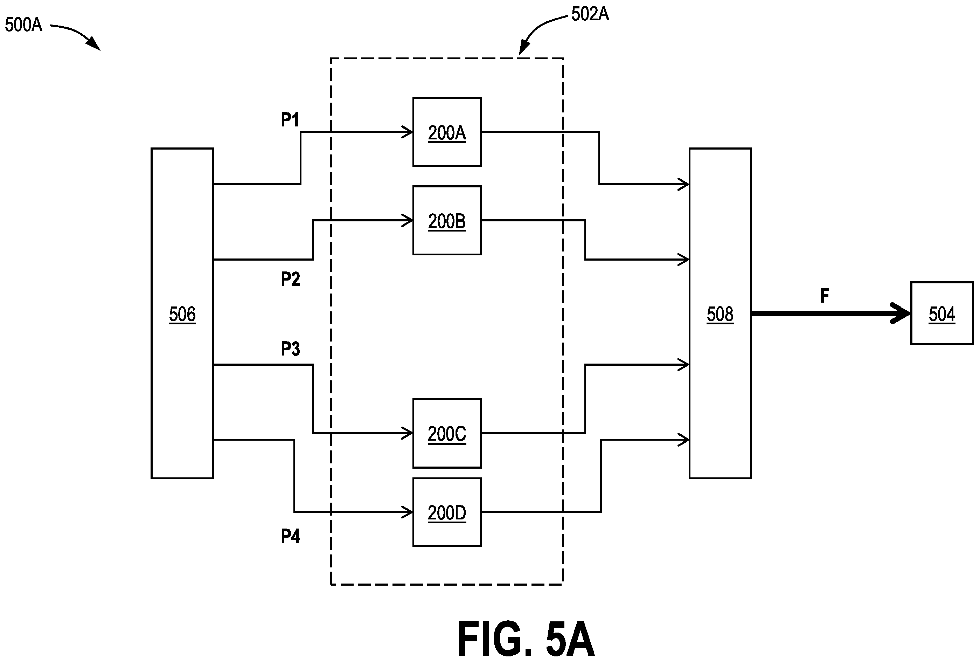

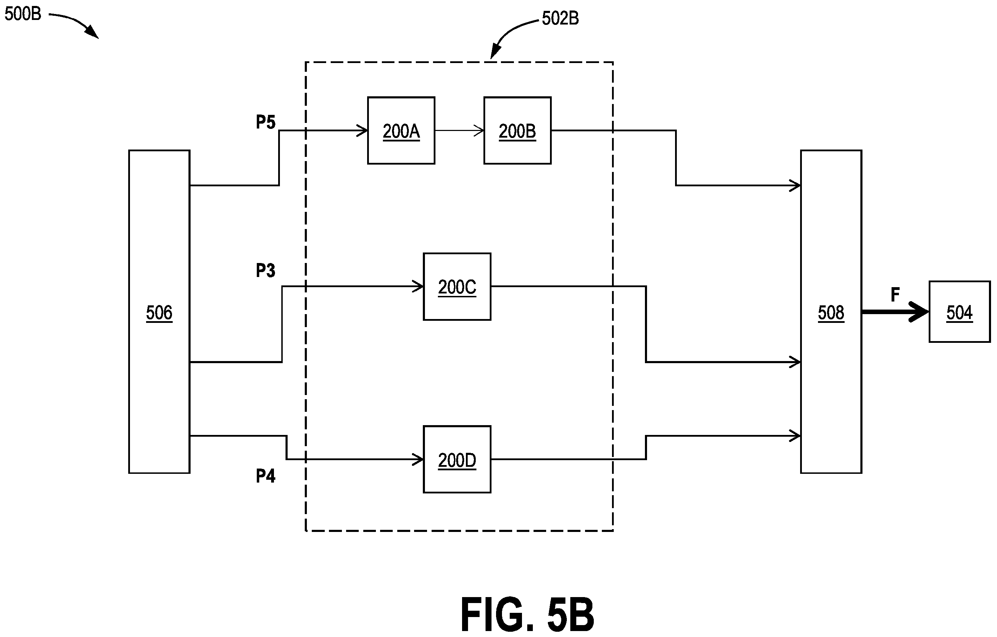

Thus, provided herein are omnidirectional pump systems comprising two or more fluid flow pumps configured to communicate a fluid flow from a fluid source to an associated device, such as an associated medical device coupled to a subject. In embodiments, the omnidirectional pump system may be configured for use in magnetic resonance environments, such as a magnetic resonance imaging (MRI) suite. For example, with reference to FIGS. 5A and 5B, omnidirectional pump systems 500A, 500B comprising an arrangement 502A, 502B of two or more pumps 200A, 200B, 200C are illustrated according to aspects of the present disclosure. As shown, the omnidirectional pump systems 500A, 500B comprise a plurality of fluid flow pumps 200A, 200B, 200C, 200D configured to communicate a fluid flow 'F' to an associated medical device 504. In embodiments, each of the pumps 200A, 200B, 200C, 200D may receive or otherwise draw a fluid flow (e.g., a first fluid flow, a second fluid flow, a third fluid flow, a fourth fluid flow, and so on). In particular embodiments, each of the pumps 200A, 200B, 200C, 200D may receive or otherwise draw a fluid flow from a fluid source 506. The fluid source 506 can include ambient air within the environment 100, but may also include one or more liquids, compressed gases, anesthetic agents, and the like.

In embodiments, the fluid inputs may travel along one or more fluid flow pathways P1, P2, P3, P4 to the medical device 504. In particular embodiments, the fluid flow pathways P1, P2, P3, P4 extend from the fluid source 506, through at least one of the pumps 200A, 200B, 200C, 200D, and to the attached medical device 504. In some embodiments, the omnidirectional pump system 500A, 500B can include a valve or other similar device 508 that receives a separate fluid flow from one or more of the pumps 200A, 200B, 200C, 200D along the fluid flow pathway P1, P2, P3, P4 and delivers a combined fluid flow 'F' to the medical device 504. However, it is contemplated that each of the pumps 200A, 200B, 200C, 200D may deliver a separate fluid flow directly to the associated medical device 504.

In the example of FIG. 5A, one or more of the pumps 200A, 200B, 200C, 200D may be connected in parallel to the valve 508 and/or medical device 504. However, it is contemplated that two or more of the pumps 200A, 200B, 200C, 200D may be connected in series. For example, as shown in FIG. 5B, a first pump 200A and a second pump 200B are connected in series, while one or more other pumps 200C, 200D are connected in parallel. In such embodiments, a fluid flow pathway P5 may be defined that extends from the fluid source 506, through at least two pumps 200A, 200B, and to the attached medical device 504.

In embodiments, the medical device 504 can be a medical device that is coupled to a subject. For example, in some embodiments, the medical device 504 may be at least one of a non-invasive blood pressure cuff, a nasal continuous positive airway pressure (CPAP) mask, a nasal pillow mask, a full-face CPAP mask, a bilevel positive airway pressure (BiPAP) mask, an anesthesia mask, and the like.

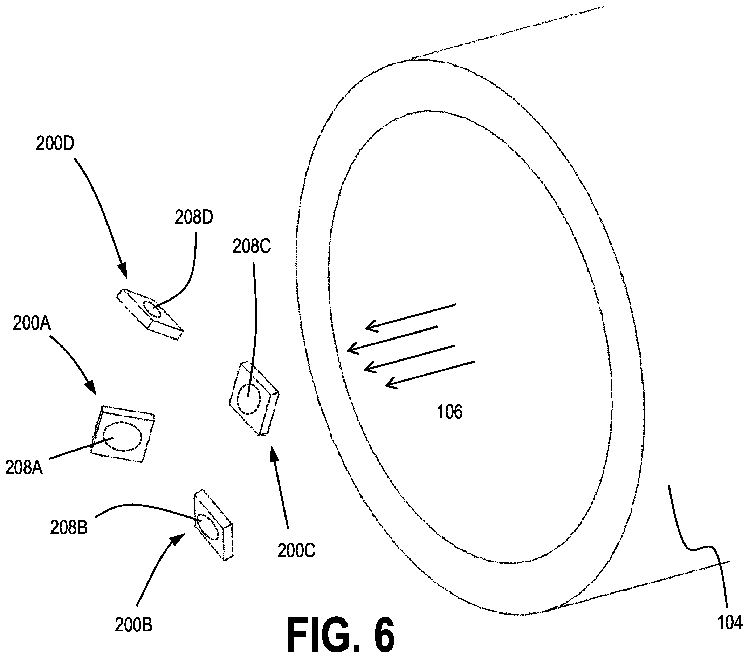

Turning to FIG. 6, a representative arrangement of multiple pumps 200A, 200B, 200C, 200D having corresponding conductive elements 208A, 208B, 208C, 208D is illustrated in proximity to a magnetic field source 104. The magnetic field source 104 may generate a magnetic field 106 within the environment 100, which is experienced by the arrangement of pumps 200A, 200B, 200C, 200D.

As shown, each of the pumps 200A, 200B, 200C, 200D can include a conductive element 208A, 208B, 208C, 208D, respectively, and may be arranged relative to the local magnetic field 106 such that at least one of a first plane and a second plane of each of the conductive elements 208A, 208B, 208C, 208D is not parallel to the direction of the magnetic field 106. That is, the pumps 200A, 200B, 200C, 200D may be arranged such that the corresponding conductive element 208A, 208B, 208C, 208D is oriented in a different direction (based on their corresponding first and/or second planes) relative to one another and/or the local magnetic field 106. In embodiments, for example, a first pump 200A may have a conductive element 208A having a first orientation, a second pump 200B may have a conductive element 208B having a second orientation that is different from the first orientation, a third pump 200C may have a conductive element 208C having a third orientation that is different from the first and second orientations, a fourth pump 200D may have a conductive element 208D having a fourth orientation that is different from the first, second, and third orientations, and so forth.

In particular embodiments, at least one of the planes of the conductive elements 208A, 208B, 208C, 208D is a maximal plane. In further embodiments, at least one of the other planes of the conductive element 208A, 208B, 208C, 208D is a minimal plane. In embodiments, the pumps 200A, 200B, 200C, 200D of the omnidirectional pump system 500A, 500B are arranged relative to one another such that the maximal plane(s) of each conductive element 208A, 208B, 208C, 208D is or are not simultaneously parallel with the direction of magnetic field 106.

In some embodiments, the pumps 200A, 200B, 200C, 200D of the omnidirectional pump system 500A, 500B may be oriented in a fixed or unfixed position relative to one another such that the maximal plane(s) of each conductive element 208A, 208B, 208C, 208D is or are not simultaneously parallel with the direction of magnetic field 106 regardless of movement and/or position of the omnidirectional pump system 500A, 500B. That is, it should be appreciated that the omnidirectional pump systems 500A, 500B may be moved between different locations (e.g., locations 108A, 108B, 108C) with an environment 100 and/or may be rotated relative to the magnetic field 106 without aligning the orientations of the conductive elements 208A, 208B, 208C, 208D with the direction of the magnetic field 106.

As such, the pumps 200A, 200B, 200C, 200D of the omnidirectional pump systems 500A, 500B may be arranged such that the maximal plane(s) of each conductive element 208A, 208B, 208C, 208D is or are not simultaneously parallel with the direction of magnetic field 106 regardless of movement and/or position of the omnidirectional pump system 500.

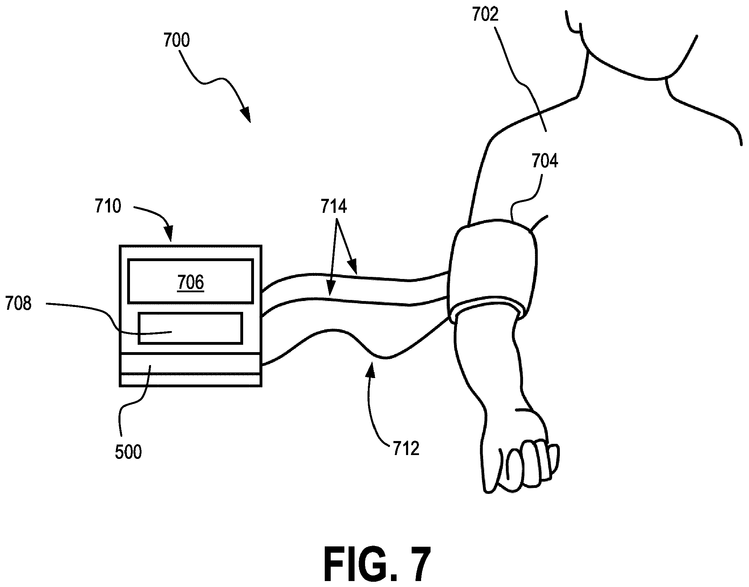

Also provided herein are medical apparatuses comprising an omnidirectional pump system 500A, 500B that is configured to produce a fluid output as described above, and a patient-worn device coupled to a patient and configured to receive the fluid output. For example, with reference to FIG. 7, a medical apparatus 700 comprising a patient-worn device 704 and an omnidirectional pump system 500 used to produce and/or regulate a fluid flow output to the patient-worn device 704 is illustrated according to aspects of the present disclosure.

In the example of FIG. 7, the medical apparatus 700 is a non-invasive blood pressure cuff configured to measure the patient's 702 blood pressure. The apparatus 700 comprises a patient-worn device 704, which can be an inflatable cuff or other inflatable device. The apparatus 700 may also include one or more sensors (not shown) configured to sense a pressure signal indicative of a pressure within the cuff and/or pulsating blood flow in an artery of the patient 702, optionally a display 706, and a controller 708 configured to operate the omnidirectional pump system 500 and/or the display 706, and configured to determine one or more physiological measurements (e.g., blood pressure, etc.) based on the signals received from the one or more sensors.

In embodiments, the omnidirectional pump system 500, the display 706, the controller 708, and/or one or more sensors (not shown) may be contained within an external housing 710. The omnidirectional pump system 500 may be operatively connected to the inflatable cuff 704 via a hose 712. In embodiments, the medical apparatus 700 further comprises one or more additional data cables 714 connecting the components of within the external housing 710 to the patient-worn device 704.

Although the medical apparatus 700 is illustrated according to one embodiment, it should be appreciated that the medical apparatus 700 could also be at least one of an MRI-compatible medical monitor, an anesthesia cart, a bedside monitor, and/or a non-invasive gas monitor. In further embodiments, the patient-worn device 704 can also be at least one of a nasal continuous positive airway pressure (CPAP) mask, a nasal pillow mask, a full-face CPAP mask, a bilevel positive airway pressure (BiPAP) mask, an anesthesia mask, and the like.

In embodiments, the medical apparatus 700 including the omnidirectional pump system 500 is particularly adapted for use in environments subjected to high magnetic fields, and may be configured to regulate delivery of a fluid flow such that the patient-worn device 704 achieves and/or maintains a target performance (e.g., a target pressure within a defined period of time).

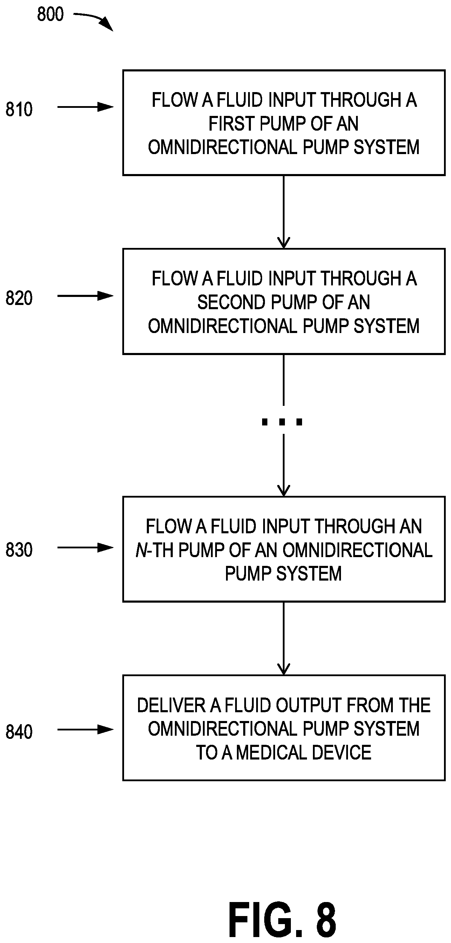

Also provided herein are methods of regulating delivery of a fluid flow to a medical device coupled to a patient. For example, with reference to FIG. 8, a method 800 of regulating the delivery of a fluid flow to a medical device (e.g., device 504, 704) is illustrated according to aspects of the present disclosure.

In a step 810, the method 800 includes flowing at least a first fluid input through a first pump of an omnidirectional pump system along a first fluid pathway to a medical device. In embodiments, the omnidirectional pump system may be an omnidirectional pump system 500, the first pump may be a pump 200, the first fluid pathway may be a fluid pathway P1, P2, P3, P4, P5, and the medical device may be an attached medical device 504, 704 as described above. In particular embodiments, the first pump 200 may comprise a fluid flow input conduit 202, a pump housing or body 204, a fluid flow output conduit 206, an actuator (not shown) configured to produce a fixed and/or variable flow rate fluid flow. As described herein, the first pump 200 also includes a conductive element 208 disposed within the pump body 204, which may be coupled to the actuator, for example.

In embodiments, the shape, size, and/or dimensions of the conductive element 208 of the first pump 200 may define at least a first plane and a second plane. In particular embodiments, at least one of the planes of the conductive element 208 of the first pump 200 is a maximal plane defining the longest straight-line distance across the conductive element 208. In further embodiments, at least one of the planes of the conductive element 208 of the first pump 200 is a minimal plane defining the shortest straight-line distance across the conductive element 208.

Accordingly, in specific embodiments, the conductive element 208 of the first pump 200 may have an orientation such that the minimal plane is parallel to the direction of a nearby magnetic field 106. In further embodiments, the conductive element 208 of the first pump 200 may have an orientation such that the maximal plane is not parallel to the direction of a nearby magnetic field 106.

In a step 820, the method 800 includes flowing at least a second fluid input through a second pump of the omnidirectional pump system along a second fluid pathway to the medical device. In embodiments, the second pump may be a pump 200, and the second fluid pathway may be a fluid pathway P1, P2, P3, P4, P5, as described above. In particular embodiments, the second pump 200 may comprise a fluid flow input conduit 202, a pump housing or body 204, a fluid flow output conduit 206, an actuator (not shown) configured to produce a fixed and/or variable flow rate fluid flow. As described herein, the second pump 200 also includes a conductive element 208 disposed within the pump body 204, which may be coupled to the actuator, for example.

In embodiments, the shape, size, and/or dimensions of the conductive element 208 of the second pump 200 may define at least a first plane and a second plane. In particular embodiments, at least one of the planes of the conductive element 208 of the second pump 200 is a maximal plane defining the longest straight-line distance across the conductive element 208. In further embodiments, at least one of the planes of the conductive element 208 of the second pump 200 is a minimal plane defining the shortest straight-line distance across the conductive element 208.

Accordingly, in specific embodiments, the conductive element 208 of the second pump 200 may have an orientation such that the minimal plane is parallel to the direction of a nearby magnetic field 106. In further embodiments, the conductive element 208 of the second pump 200 may have an orientation such that the maximal plane is not parallel to the direction of a nearby magnetic field 106.

In still further embodiments, the conductive element 208 of the second pump 200 may have an orientation such that its maximal plane is not parallel to the maximal plane of the conductive element 208 of the first pump 200. In other words, the second pump 200 may have an orientation that is different from the orientation of the first pump. In specific embodiments, the first and second pumps 200 may be arranged such that the maximal planes of the corresponding conductive elements 208 are not simultaneously parallel to the direction of an associated magnetic field.

In a step 830, the method 800 includes flowing at least a third fluid input through one or more additional pumps of the omnidirectional pump system along one or more additional fluid pathway to the medical device. In embodiments, the omnidirectional pump system can comprise *N* number of pumps 200, and *N* number of fluid pathways P1, P2, P3, P4, P5, including but not limited to, at least four pumps, at least 5 pumps, at least 6 pumps, and at least 10 pumps. In particular embodiments, the *N*-th pump 200 may comprise a fluid flow input conduit 202, a pump housing or body 204, a fluid flow output conduit 206, an actuator (not shown) configured to produce a fixed and/or variable flow rate fluid flow. As described herein, the N-th pump 200 also includes a conductive element 208 disposed within the pump body 204, which may be coupled to the actuator, for example.

In embodiments, the shape, size, and/or dimensions of the conductive element 208 of the N-th pump 200 may define at least a first plane and a second plane. In particular embodiments, at least one of the planes of the conductive element 208 of the *N*-th pump 200 is a maximal plane defining the longest straight-line distance across the conductive element 208. In further embodiments, at least one of the planes of the conductive element 208 of the *N*-th pump 200 is a minimal plane defining the shortest straight-line distance across the conductive element 208.

Accordingly, in specific embodiments, the conductive element 208 of the *N*-th pump 200 may have an orientation such that the minimal plane is parallel to the direction of a nearby magnetic field 106. In further embodiments, the conductive element 208 of the *N*-th pump 200 may have an orientation such that the maximal plane is not parallel to the direction of a nearby magnetic field 106.

In still further embodiments, the conductive element 208 of the *N*-th pump 200 may have an orientation such that its maximal plane is not parallel to the maximal planes of the conductive elements 208 of the first or second pumps 200. In other words, the *N*-th pump 200 may have an orientation that is different from the orientation of at least the first and second pumps. In specific embodiments, each of the pumps 200 may be arranged such that the maximal planes of the corresponding conductive elements 208 are not simultaneously parallel to the direction of an associated magnetic field.

In a step 840, the method 800 includes delivering a fluid output (e.g., fluid flow 'F') to a medical device (e.g., medical device 504, 704) that is coupled to a patient (e.g., patient 702). In embodiments, the fluid output achieves a target flow rate within a target period of time. For example, in some embodiments, the fluid output can have an output flowrate such that the medical device 504, 704 coupled to the patient 702 achieves a target pressure within a target duration.

In embodiments, the target pressure for the medical device 504, 704 may be from about 80 mmHg to about 300 mmHg, including at least about 100 mmHg, at least about 110 mmHg, at least about 120 mmHg, at least about 130 mmHg, at least about 140 mmHg, at least about 150 mmHg, at least about 160 mmHg, at least about 170 mmHg, at least about 180 mmHg, at least about 190 mmHg, at least about 200 mmHg, at least about 210 mmHg, at least about 220 mmHg, at least about 230 mmHg, at least about 240 mmHg, at least about 250 mmHg, at least about 260 mmHg, at least about 270 mmHg, at least about 280 mmHg, at least about 290 mmHg, and at least about 300 mmHg.

In further embodiments, the target duration for the medical device 504, 704 to achieve the target pressure may be less than about 30 seconds, including less than about 25 seconds, less than about 20 seconds, less than about 15 seconds, and less than about 10 seconds.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

Other implementations are within the scope of the following claims.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications as long as they fall under the scope of the appended claims.

More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein.

## Claims

1. An omnidirectional pump system (500A, 500B) for use in a magnetic resonance environment, the pump system comprising:
a first pump (200A) configured to communicate a first fluid flow from a fluid source (506) to an associated medical device (504), the first pump including a conductive element (208A) having a first plane; **characterized by**:
a second pump (200B) configured to communicate a second fluid flow from the fluid source to the medical associated device concurrently with the first pump, the second pump including a conductive element (208B) having a second plane;
wherein the first pump and the second pump are arranged such that the first plane and the second plane are not simultaneously parallel to an associated magnetic field (106) in proximity to the omnidirectional pump system.

2. The omnidirectional pump system of claim 1, wherein the first pump and the second pump are arranged such that the first plane and the second plane cannot be not simultaneously be parallel to an associated magnetic field in proximity to the omnidirectional pump system.

3. The omnidirectional pump system of claim 1, wherein the associated magnetic field in proximity to the omnidirectional pump system is perpendicular to at least one of the first plane of the conductive element of the first pump and the second plane of the conductive element of the second pump.

4. The omnidirectional pump system of claim 1, wherein the associated magnetic field has a magnetic flux density of at least 1,000 gauss, and wherein the first pump and the second pump are rated for use in the associated magnetic field.

5. The omnidirectional pump system of claim 1, further comprising a third pump (200C) configured to communicate a third fluid flow from the fluid source to the associated medical device with the first and second pumps, the third pump including a conductive element (208C) having a third plane, wherein the first, second, and third pumps are arranged such that the first, second, and third planes are not simultaneously parallel to the associated magnetic field in proximity to the omnidirectional pump system.

6. The omnidirectional pump system of claim 1, wherein the first pump is at least one of a rolling magnet pump, a stator rotation pump, and a piezoelectric pump; and
wherein the second pump is at least one of a rolling magnet pump, a stator rotation pump, and a piezoelectric pump.

7. The omnidirectional pump system of claim 1, wherein the associated medical device is a non-invasive blood pressure cuff, and the first and second fluid flows comprise air.

8. The omnidirectional pump system of claim 1, wherein the associated medical device is a respiratory mask, and at least one of the first and second fluid flows comprises an anesthetic agent.

9. The omnidirectional pump system of claim 1, wherein the omnidirectional pump system is movable relative to the associated magnetic field while the first plane of the conductive element of the first pump is fixed relative to the second plane of the conductive element of the second pump.

10. The omnidirectional pump system of claim 1, wherein each of the conductive element are actuated during operation of the corresponding pump.

11. A medical apparatus (700) comprising an omnidirectional pump system (500A, 500B) configured to produce a fluid output and a patient-worn device (704) configured to receive the fluid output, the patient-worn device being coupled to a patient, wherein the omnidirectional pump system is configured as defined in claim 1.

12. The medical apparatus of claim 11, wherein the medical apparatus is at least one of: an MRI-compatible medical monitor; an anesthesia cart; a bedside monitor; a non-invasive blood pressure monitor; and a non-invasive gas monitor.

13. A method (800) of regulating delivery of a fluid flow to a medical device (504, 704) coupled to a patient, the method comprising:
flowing (801) a first fluid input though at least a first pump (200A) of an omnidirectional pump system (500A, 500B) along a first fluid pathway to the medical device; and
flowing (820) a second fluid input through at least a second pump (200B) of the omnidirectional pump system along a second fluid pathway to the medical device;
wherein the first pump includes a conductive element having a first plane, the second pump includes a conductive element (208B) having a second plane, and the first pump and second pump are arranged such that the first plane and the second plane are not simultaneously parallel to an associated magnetic field (106) in proximity to the medical device.

14. The method of claim 13, wherein the omnidirectional pump system is movable relative to the associated magnetic field while the first plane of the conductive element of the first pump is fixed relative to the second plane of the conductive element of the second pump.

15. The method of claim 13, wherein the first plane is defined by the longest straight-line distance through the conductive element 208 of the first pump, and the second plane is defined by the longest straight-line distance through the conductive element of the second pump.

## Patentansprüche

1. Omnidirektionales Pumpensystem (500A, 500B) zur Verwendung in einer Magnetresonanzumgebung, wobei das Pumpensystem umfasst:
eine erste Pumpe (200A), die konfiguriert ist, um einen ersten Fluidstrom von einer Fluidquelle (506) mit einer zugeordneten medizinischen Vorrichtung (504) zu verbinden, wobei die erste Pumpe ein leitfähiges Element (208A) beinhaltet, das eine erste Ebene aufweist; **gekennzeichnet durch**:
eine zweite Pumpe (200B), die konfiguriert ist, um gleichzeitig mit der ersten Pumpe einen zweiten Fluidstrom von der Fluidquelle mit der zugeordneten medizinischen Vorrichtung zu verbinden, wobei die zweite Pumpe ein leitfähiges Element (208B) beinhaltet, das eine zweite Ebene aufweist;
wobei die erste Pumpe und die zweite Pumpe angeordnet sind, sodass die erste Ebene und die zweite Ebene nicht gleichzeitig parallel zu einem zugeordneten Magnetfeld (106) in der Nähe des omnidirektionalen Pumpensystems sind.

2. Omnidirektionales Pumpensystem nach Anspruch 1, wobei die erste Pumpe und die zweite Pumpe angeordnet sind, sodass die erste Ebene und die zweite Ebene nicht gleichzeitig parallel zu einem zugeordneten Magnetfeld in der Nähe des omnidirektionalen Pumpensystems sein können.

3. Omnidirektionales Pumpensystem nach Anspruch 1, wobei das zugeordnete Magnetfeld in der Nähe des omnidirektionalen Pumpensystems senkrecht zu mindestens einer von der ersten Ebene des leitfähigen Elements der ersten Pumpe und der zweiten Ebene des leitfähigen Elements der zweiten Pumpe ist.

4. Omnidirektionales Pumpensystem nach Anspruch 1, wobei das zugeordnete Magnetfeld eine magnetische Flussdichte von mindestens 1.000 Gauß aufweist und wobei die erste Pumpe und die zweite Pumpe zur Verwendung in dem zugeordneten Magnetfeld ausgelegt sind.

5. Omnidirektionales Pumpensystem nach Anspruch 1, weiter umfassend eine dritte Pumpe (200C), die konfiguriert ist, um einen dritten Fluidstrom von der Fluidquelle mit der zugeordneten medizinischen Vorrichtung mit der ersten und zweiten Pumpe zu verbinden, wobei die dritte Pumpe ein leitfähiges Element (208C) beinhaltet, das eine dritte Ebene aufweist, wobei die erste, zweite und dritte Pumpe angeordnet sind, sodass die erste, zweite und dritte Ebene nicht gleichzeitig parallel zum zugeordneten Magnetfeld in der Nähe des omnidirektionalen Pumpensystems sind.

6. Omnidirektionales Pumpensystem nach Anspruch 1, wobei die erste Pumpe mindestens eine von einer Rollmagnetpumpe, einer Statorrotationspumpe oder einer piezoelektrischen Pumpe ist; und
wobei die zweite Pumpe mindestens eine von einer Rollmagnetpumpe, einer Statorrotationspumpe oder einer piezoelektrischen Pumpe ist.

7. Omnidirektionales Pumpensystem nach Anspruch 1, wobei die zugeordnete medizinische Vorrichtung eine nicht-invasive Blutdruckmanschette ist und der erste und zweite Fluidstrom Luft umfassen.

8. Omnidirektionales Pumpensystem nach Anspruch 1, wobei die zugeordnete medizinische Vorrichtung eine Beatmungsmaske ist und mindestens einer von dem ersten und zweiten Fluidstrom ein Anästhetikum umfasst.

9. Omnidirektionales Pumpensystem nach Anspruch 1, wobei das omnidirektionale Pumpensystem in Bezug auf das zugeordnete Magnetfeld beweglich ist, während die erste Ebene des leitfähigen Elements der ersten Pumpe in Bezug zur zweiten Ebene des leitfähigen Elements der zweiten Pumpe feststehend ist.

10. Omnidirektionales Pumpensystem nach Anspruch 1, wobei jedes der leitfähigen Elemente während eines Betriebs der entsprechenden Pumpe betätigt wird.

11. Medizinische Einrichtung (700), die ein omnidirektionales Pumpensystem (500A, 500B), das konfiguriert ist, um einen Fluidaustrag zu erzeugen, und eine vom Patienten getragene Vorrichtung (704) umfasst, die konfiguriert ist, um den Fluidaustrag zu empfangen, die vom Patienten getragene Vorrichtung ist mit einem Patienten gekoppelt, und wobei das omnidirektionale Pumpensystem wie in Anspruch 1 definiert konfiguriert ist.

12. Medizinische Einrichtung nach Anspruch 11, wobei die medizinische Einrichtung mindestens eines ist von: einem MRT-kompatiblen medizinischen Monitor; einem Anästhesiewagen; einem bettseitigen Monitor; einem nicht-invasives Blutdruckmonitor; und einem nicht-invasives Gasmonitor.

13. Verfahren (800) zum Regeln der Zufuhr eines Fluidstroms zu einer mit einem Patienten gekoppelten medizinischen Vorrichtung (504, 704), wobei das Verfahren umfasst:
Strömen lassen (801) eines ersten Fluideingangs durch mindestens eine erste Pumpe (200A) eines omnidirektionalen Pumpensystems (500A, 500B) entlang eines ersten Fluidwegs zur medizinischen Vorrichtung; und
Strömen lassen (820) eines zweiten Fluideingangs durch mindestens eine zweite Pumpe (200B) des omnidirektionalen Pumpensystems entlang eines zweiten Fluidwegs zur medizinischen Vorrichtung;
wobei die erste Pumpe ein leitfähiges Element beinhaltet, das eine erste Ebene aufweist, die zweite Pumpe ein leitfähiges Element (208B) beinhaltet, das eine zweite Ebene aufweist und die erste Pumpe und zweite Pumpe angeordnet sind, sodass die erste Ebene und die zweite Ebene nicht gleichzeitig parallel zu einem zugeordneten Magnetfeld (106) in der Nähe der medizinischen Vorrichtung sind.

14. Verfahren nach Anspruch 13, wobei das omnidirektionale Pumpensystem in Bezug auf das zugeordnete Magnetfeld beweglich ist, während die erste Ebene des leitfähigen Elements der ersten Pumpe in Bezug zur zweiten Ebene des leitfähigen Elements der zweiten Pumpe feststehend ist.

15. Verfahren nach Anspruch 13, wobei die erste Ebene durch die längste geradlinige Entfernung durch das leitfähige Element 208 der ersten Pumpe hindurch definiert ist und die zweite Ebene durch die längste geradlinige Entfernung durch das leitfähige Element der zweiten Pumpe hindurch definiert ist.

## Revendications

1. Système de pompe omnidirectionnel (500A, 500B) destiné à être utilisé dans un environnement de résonance magnétique, le système de pompe comprenant :
une première pompe (200A) configurée pour acheminer un premier flux de fluide depuis une source de fluide (506) vers un dispositif médical associé (504), la première pompe incluant un élément conducteur (208A) présentant un premier plan ; **caractérisée par** :
une deuxième pompe (200B) configurée pour communiquer un deuxième flux de fluide depuis la source de fluide au dispositif médical associé simultanément avec la première pompe, la deuxième pompe incluant un élément conducteur (208B) présentant un deuxième plan ;
dans lequel la première pompe et la deuxième pompe sont agencées de telle sorte que le premier plan et le deuxième plan ne soient pas simultanément parallèles à un champ magnétique associé (106) à proximité du système de pompe omnidirectionnel.

2. Système de pompe omnidirectionnel selon la revendication 1, dans lequel la première pompe et la deuxième pompe sont agencées de telle sorte que le premier plan et le deuxième plan ne peuvent pas être simultanément parallèles à un champ magnétique associé à proximité du système de pompe omnidirectionnel.

3. Système de pompe omnidirectionnel selon la revendication 1, dans lequel le champ magnétique associé à proximité du système de pompe omnidirectionnel est perpendiculaire à au moins l'un des plans du premier élément conducteur de la première pompe et du deuxième plan de l'élément conducteur de la deuxième pompe.

4. Système de pompe omnidirectionnel selon la revendication 1, dans lequel le champ magnétique associé présente une densité de flux magnétique d'au moins 1.000 gauss, et dans lequel la première pompe et la deuxième pompe sont conçues pour être utilisées dans le champ magnétique associé.

5. Système de pompe omnidirectionnel selon la revendication 1, comprenant en outre une troisième pompe (200C) configurée pour communiquer un troisième flux de fluide depuis la source de fluide au dispositif médical associé avec les première et deuxième pompes, la troisième pompe incluant un élément conducteur (208C) présentant un troisième plan, dans lequel les première, deuxième et troisième pompes sont agencées de telle sorte que les premier, deuxième et troisième plans ne soient pas simultanément parallèles au champ magnétique associé à proximité du système de pompe omnidirectionnel.

6. Système de pompe omnidirectionnel selon la revendication 1, dans lequel la première pompe est au moins l'une d'une pompe à aimant roulant, d'une pompe à rotation de stator et d'une pompe piézoélectrique ; et
dans lequel la seconde pompe est au moins une pompe à aimant roulant, une pompe à rotation de stator et une pompe piézoélectrique.

7. Système de pompe omnidirectionnel selon la revendication 1, dans lequel le dispositif médical associé est un brassard de pression artérielle non invasif, et les premier et deuxième flux de fluide comprennent de l'air.

8. Système de pompe omnidirectionnel selon la revendication 1, dans lequel le dispositif médical associé est un masque respiratoire, et au moins l'un des premier et deuxième flux de fluide comprend un agent anesthésique.

9. Système de pompe omnidirectionnel selon la revendication 1, dans lequel le système de pompe omnidirectionnel est mobile par rapport au champ magnétique associé tandis que le premier plan de l'élément conducteur de la première pompe est fixe par rapport au deuxième plan de l'élément conducteur de la deuxième pompe.

10. Système de pompe omnidirectionnel selon la revendication 1, dans lequel chacun des éléments conducteurs est actionné pendant le fonctionnement de la pompe correspondante.

11. Appareil médical (700) comprenant un système de pompe omnidirectionnel (500A, 500B) configuré pour produire un débit de fluide et un dispositif porté par le patient (704) configuré pour recevoir le débit de fluide, le dispositif porté par le patient étant couplé à un patient, dans lequel le système de pompe omnidirectionnel est configuré comme défini dans la revendication 1.

12. Appareil médical selon la revendication 11, dans lequel l'appareil médical est au moins l'un des suivants : un moniteur médical compatible avec l'IRM ; un chariot d'anesthésie ; un moniteur de chevet ; un moniteur de pression artérielle non invasif ; et un moniteur de gaz non invasif.

13. Procédé (800) de régulation du débit d'un fluide alimentant un dispositif médical (504, 704) relié à un patient, le procédé comprenant :
l'écoulement (801) d'une première entrée de fluide à travers au moins une première pompe (200A) d'un système de pompe omnidirectionnel (500A, 500B) le long d'une première voie de fluide vers le dispositif médical ; et
l'écoulement (820) d'une deuxième entrée de fluide à travers au moins une deuxième pompe (200B) du système de pompe omnidirectionnel le long d'une deuxième voie de fluide vers le dispositif médical ;
dans lequel la première pompe inclut un élément conducteur présentant un premier plan, la deuxième pompe inclut un élément conducteur (208B) présentant un deuxième plan, et la première pompe et la deuxième pompe sont agencées de telle sorte que le premier plan et le deuxième plan ne soient pas simultanément parallèles à un champ magnétique associé (106) à proximité du dispositif médical.

14. Procédé selon la revendication 13, dans lequel le système de pompe omnidirectionnel est mobile par rapport au champ magnétique associé tandis que le premier plan de l'élément conducteur de la première pompe est fixe par rapport au deuxième plan de l'élément conducteur de la deuxième pompe.

15. Procédé selon la revendication 13, dans lequel le premier plan est défini par la plus grande distance en ligne droite à travers l'élément conducteur 208 de la première pompe, et le deuxième plan est défini par la plus grande distance en ligne droite à travers l'élément conducteur de la deuxième pompe.
